(19) Europäisches Patentamt
European Patent Office
Office européen des brevets

(11) Publication number: **0 172 968 A1**

(12) **EUROPEAN PATENT APPLICATION**

(21) Application number: **84307285.1**

(22) Date of filing: **23.10.84**

(51) Int. Cl.⁴: **C 07 D 285/18**, C 07 D 417/12, A 61 K 31/54

(30) Priority: **28.10.83 GB 8328907**

(43) Date of publication of application: **05.03.86 Bulletin 86/10**

(84) Designated Contracting States: **AT BE CH DE FR GB IT LI LU NL SE**

(71) Applicant: **SMITH KLINE & FRENCH LABORATORIES LIMITED, Mundells, Welwyn Garden City Hertfordshire, AL7 1EY (GB)**

(72) Inventor: **Durant, Graham John, Pengelly 15A Briary Wood Lane, Welwyn Herstfordshire AL6 0TE (GB)**
Inventor: **Brown, Thomas Henry, 17 Godfries Close, Tewin Hertfordshire (GB)**

(74) Representative: **Denerley, Paul Millington, Dr. et al, Smith Kline & French Laboratories Ltd Patent Department Mundells, Welwyn Garden City Hertfordshire AL7 1EY (GB)**

(54) **Aminothiadiazine derivatives as histamine H2-antagonists.**

(57) Compounds of the formula (I):

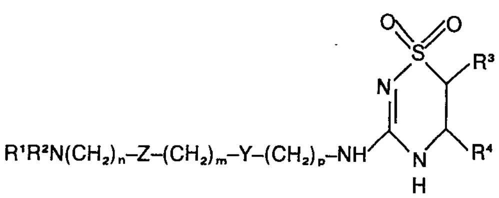

(I)

and salts thereof, wherein $R^1$ and $R^2$ are hydrogen, alkyl, substituted alkyl or are joined; n is 1 to 6; Z is 1,3-phenylene, 1,4-phenylene, 2,4-pyridyl wherein the $R^1R^2N(CH_2)_n$ group is in the 4-position; m is zero or one; Y is oxygen, sulphur or methylene; p is two, three or four; and $R^3$ and $R^4$ are independently hydrogen, $C_{1-6}$alkyl, aryl, aryl($C_{1-6}$)alkyl or pyridyl($C_{1-6}$)alkyl, or $R^3$ and $R^4$ together with the carbon atoms to which they are attached form a benzene ring optionally substituted by $C_{1-6}$alkyl are described as histamine $H_2$-antagonists. Their use in pharmaceutical compositions is described, as are processes for their preparation.

EP 0 172 968 A1

# CHEMICAL COMPOUNDS

This invention relates to thiadiazine derivatives, processes for their preparation, pharmaceutical compositions containing them and their use as histamine $H_2$-antagonists.

Histamine, a physiologically active compound endogenous in mammals, exerts its action by interacting with certain sites called receptors. One type of receptor is known as a histamine $H_1$-receptor (Ash and Schild, Brit. J. Pharmac. Chemother. 27 427 (1966)) and the actions of histamine mediated through these receptors are blocked by drugs commonly called "antihistamines" (histamine $H_1$-antagonists) a common example of which is mepyramine. A second type of histamine receptor is known as the $H_2$-receptor (Black et al. Nature 1972, 236, 385). These receptors are not blocked by mepyramine but are blocked by burimamide. Compounds which block these histamine $H_2$-receptors are called histamine $H_2$-antagonists.

Histamine $H_2$-antagonists are useful in treating disease conditions caused by the biological effects of histamine mediated through $H_2$-receptors, for example, as inhibitors of gastric acid secretion, in the treatment of inflammation mediated through histamine $H_2$-receptors and as agents which act on the cardiovascular system, for example, as inhibitors of effects of histamine on blood pressure mediated through histamine $H_2$-receptors.

Cimetidine is an example of a histamine $H_2$-antagonist. Cimetidine has been shown to be useful in the treatment of duodenal, gastric, recurrent and stomal ulceration, and reflux oesophagitis and in the management of patients who are at high risk from haemorrhage of the upper gastrointestinal tract.

In some physiological conditions the biological actions of histamine are mediated through both histamine $H_1$- and $H_2$-receptors and blockade of both types of receptors is useful. These conditions include inflammation mediated by histamine, for example skin inflammation, and those hypersensitivity responses due to the action of histamine at $H_1$- and $H_2$-receptors, for example allergies.

Accordingly the present invention provides compounds of the formula (I) :-

$R^1R^2N(CH_2)_n$-Z-$(CH_2)_m$-Y-$(CH_2)_p$-NH 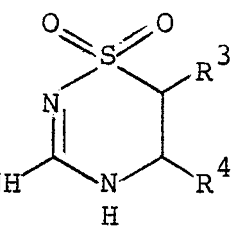 (I)

and pharmaceutically acceptable salts thereof, wherein

$R^1$ and $R^2$ are independently hydrogen, $C_{1-6}$alkyl, aryl($C_{1-6}$)alkyl, heteroaryl($C_{1-6}$)alkyl, $C_{3-10}$cycloalkyl, hydroxy($C_{2-6}$)alkyl, or halo-($C_{2-6}$)alkyl, (wherein said hydroxy and halo groups are not substituted on the carbon atom adjacent to the nitrogen atom); or

$R^1$ and $R^2$ together represent $-(CH_2)_q-$ wherein q is 4 to 7, to form together with the nitrogen atom to which they are attached a 5-8 membered saturated ring;

n is an integer from 1 to 6;

Z is 1,3-phenylene, 1,4-phenylene, 2,4-pyridyl (wherein the $R^1R^2N(CH_2)_n$ group is in the 4-position);

m is zero or one ;

Y is oxygen, sulphur or methylene;

p is two, three or four; and

$R^3$ and $R^4$ are independently hydrogen, $C_{1-6}$alkyl, aryl, aryl($C_{1-6}$)alkyl or pyridyl($C_{1-6}$)alkyl, or $R^3$ and $R^4$ together with the carbon atoms to which they are attached form a benzene ring optionally substituted by $C_{1-6}$alkyl.

When used herein alkyl means groups that are either straight-chained or branched. In general preferred alkyl groups are methyl and ethyl.

Suitably in the definitions of $R^1$, $R^2$, $R^3$ and $R^4$ aryl is phenyl, aryl($C_{1-6}$)alkyl is phenyl($C_{1-6}$)alkyl and heteroaryl($C_{1-6}$)alkyl is furanyl($C_{1-6}$)alkyl or thienyl-($C_{1-6}$)alkyl.

Suitably $R^1$ is aryl($C_{1-6}$)alkyl for example benzyl or phenethyl, heteroaryl($C_{1-6}$)alkyl for example furanyl-($C_{1-6}$)alkyl such as furanylmethyl or thienyl($C_{1-6}$)alkyl such as thienylmethyl, halo($C_{2-6}$)alkyl for example 2,2,2-trifluoroethyl, or $C_{3-10}$cycloalkyl for example cyclohexyl. More suitably $R^1$ is $C_{1-6}$alkyl, for example methyl, ethyl or propyl.

Suitably $R^2$ is hydrogen or $C_{1-6}$alkyl, for example methyl, ethyl or propyl.

Suitably $R^1$ and $R^2$ have the same value, for example they both are methyl or they are both ethyl. In another suitable aspect $R^1$ and $R^2$ together with the nitrogen atom to which they are attached form a pyrrolidino or piperidino ring.

Preferably n is one.

Suitably Z is 1,3-phenylene. Suitably Z is 2,4-pyridyl. Favourably when Z is 1,3-phenylene, m is zero

and preferably Y is oxygen. Favourably when Z is 2,4-pyridyl m is one and preferably Y is sulphur. In an alternative favourable aspect when Z is 2,4-pyridyl, m is zero and preferably Y is oxygen.

Suitably m and p when added together equals three, that is suitably p is two when m is one, and suitably p is three when m is zero.

Examples of favoured groups $R^1R^2N(CH_2)_n$-Z-$(CH_2)_m$-Y-$(CH_2)_p$ include :

4-dimethylaminomethylpyrid-2-ylmethylthioethyl,
4-piperidinomethylpyrid-2-ylmethylthioethyl,
4-dimethylaminomethylpyrid-2-yloxypropyl,
4-piperidinomethylpyrid-2-yloxypropyl,
3-dimethylaminomethylphenoxypropyl,
3-piperidinomethylphenoxypropyl,
3-dimethylaminomethylphenylmethylthioethyl or
3-piperidinomethylphenylmethylthioethyl.

In one aspect of this invention $R^3$ and $R^4$ are independently hydrogen, $C_{1-6}$alkyl, aryl, aryl($C_{1-6}$)alkyl or pyridyl($C_{1-6}$)alkyl.

Suitably $R^3$ is hydrogen, $C_{1-6}$alkyl for example methyl, ethyl, propyl or butyl, aryl($C_{1-6}$)alkyl for example benzyl, phenethyl, or is pyridylmethyl.

Suitably $R^4$ is hydrogen or $C_{1-6}$alkyl for example methyl or ethyl.

In a preferred aspect $R^3$ and $R^4$ are both hydrogen.

In a further aspect $R^3$ and $R^4$ together with the carbon atoms to which they are attached form a benzene ring.

The compounds of the formula (I) are depicted in the 4(H) tautomeric form, and exist in equilibrium with the 2(H) tautomeric form and the tautomeric form with an exocyclic double bond :-

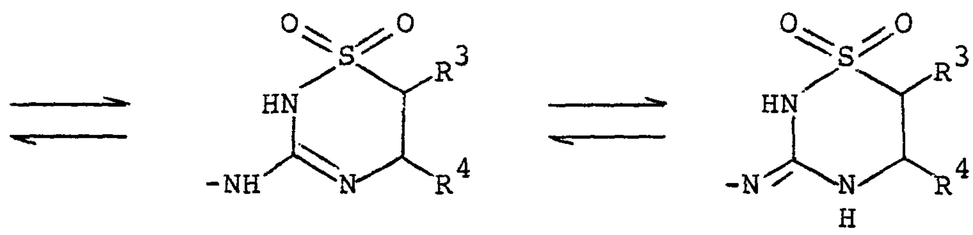

The present application covers all isomeric and tautomeric forms, and mixtures thereof, of the compounds of the formula (I).

Specific compounds of this invention include :

3-[3-(3-(piperidinomethyl)phenoxy)propylamino]-5,6-dihydro-1,2,4-thiadiazine-1,1-dioxide and pharmaceutically acceptable salts thereof;

3-[3-(3-(piperidinomethyl)phenoxy)propylamino]-1,2,4-benzothiadiazine-1,1-dioxide and pharmaceutically acceptable salts thereof;

3-[3-(4-(dimethylaminomethyl)pyrid-2-yloxy)propylamino]-1,2,4-benzothiadiazine-1,1-dioxide and pharmaceutically acceptable salts thereof;

3-[3-(4-(piperidinomethyl)pyrid-2-yloxy)propylamino]-1,2,4-benzothiadiazine-1,1-dioxide and pharmaceutically acceptable salts thereof;

3-[3-(4-(piperidinomethyl)pyrid-2-yloxy)propylamino]-5,6-dihydro-1,2,4-thiadiazine-1,1-dioxide and pharmaceutically acceptable salts thereof;

3-[2-(4-(dimethylaminomethyl)pyrid-2-ylmethylthio)ethylamino]-1,2,4-benzothiadiazine-1,1-dioxide and pharmaceutically acceptable salts thereof; and

3-[2-(4-(dimethylaminomethyl)pyrid-2-ylmethylthio)ethylamino]-5,6-dihydro-1,2,4-thiadiazine-1,1-dioxide and pharmaceutically acceptable salts thereof.

The activity of the compounds of formula (I) as histamine $H_2$-antagonists can be demonstrated by their ability to inhibit histamine-stimulated secretion of gastric acid from the lumen-perfused stomachs of rats anaesthetised with urethane, and to reverse histamine-induced inhibition of contractions of the isolated rat uterus. These are actions of histamine which, according to Ash and Schild, Brit. J. Pharmac. Chemother. 27 247 (1966), are not mediated by histamine $H_1$-receptors.

The histamine $H_2$-antagonist activity of the compounds can also be demonstrated by the inhibition of histamine-stimulated acid secretion in the Heidenhain Pouch Dog, the inhibition of histamine-induced tachycardia in the isolated guinea pig right atrium and the inhibition of histamine-induced vasodilatation in the anaesthetised cat.

The measurement of inhibition of histamine-stimulated secretion of gastric acid from the lumen-perfused stomachs of rats anaesthetised with urethane, and the measurement of inhibition of histamine-induced tachycardia in the isolated guinea pig right atrium, are detailed in European Patent Application Publication No 49173.

To illustrate the level of activity of the compounds of the invention we have determined that where tested the products of the Examples have $ED_{50}$ values in the lumen-perfused rat test of less than one micromol $kg^{-1}$ i.v. and $pA_2$ values in the guinea pig atrium test of more than six.

In order to use compounds of formula (I) or pharmaceutically acceptable salts thereof for medical purposes, they are normally formulated in accordance with standard pharmaceutical practice as pharmaceutical compositions.

The invention further provides pharmaceutical compositions comprising a compound of formula (I) above or a pharmaceutically acceptable salt thereof together with a pharmaceutically acceptable carrier.

Compounds of formula (I) and their pharmaceutically acceptable salts may be administered orally, parenterally, cutaneously or rectally.

Compounds of formula (I) and their pharmaceutically acceptable salts which are active when given orally can be formulated as syrups, tablets, capsules and lozenges. A syrup formulation will generally consist of a suspension or solution of the compound or salt in a suitable liquid carrier for example, ethanol, glycerine or water with a flavouring or colouring agent. Where the composition is in the form of a tablet, any suitable pharmaceutical carrier routinely used for preparing solid formulations may be used. Examples of such carriers include magnesium stearate, starch, lactose, sucrose and cellulose.

Typical parenteral compositions consist of a solution or suspension of the compound or pharmaceutically acceptable salt thereof in a sterile aqueous carrier or parenterally acceptable oil.

Typical compositions for administration to the skin include lotions and creams in which the compound of formula (I) or pharmaceutically acceptable salt thereof is contained in a liquid vehicle.

A typical suppository formulation comprises a compound of formula (I) or a pharmaceutically acceptable salt thereof which is active when administered in this way, with a binding and/or lubricating agent such as gelatin or cocoa butter or other low melting vegetable waxes or fats.

Preferably the composition is in unit dose form such as a tablet or capsule so that the patient may administer to himself a single dose.

Each dosage unit for oral administration contains preferably from 15 to 250 mg (and for parenteral administration contains preferably 0.5 to 25 mg) of a compound of the formula (I) or a pharmaceutically acceptable salt thereof calculated as the free base.

The invention also provides a method of blocking histamine $H_2$-receptors which comprises administering to an animal an effective amount to block said receptors of a compound of the formula (I) or pharmaceutically acceptable salt thereof.

The pharmaceutical compositions of this invention will normally be administered to a subject for the treatment of peptic ulcers and other conditions caused or exacerbated by gastric acidity in the same general manner as that employed for known histamine $H_2$-antagonists, due allowance being made in terms of dose levels for the potency of the compound of the present invention relative to known histamine $H_2$-antagonists. Thus an adult patient will receive an oral dose of between 15 mg and 1500 mg and preferably between 20 mg and 250 mg or an intravenous, subcutaneous, or intramuscular dose of between 0.5 mg and 150 mg, and preferably between 1.0 mg and 20 mg of compound of formula (I) or pharmaceutically

acceptable salt thereof calculated as the free base, the composition being administered 1 to 6 times per day.

In a further aspect of this invention the compounds of the formula (I) and pharmaceutically acceptable salts thereof may be prepared by a process which comprises :

a) reacting a compound of the formula (II) with a compound of the formula (III) :

$$R^1R^2N(CH_2)_n-Z-(CH_2)_m-Y-(CH_2)_p-NH_2 \qquad (II)$$

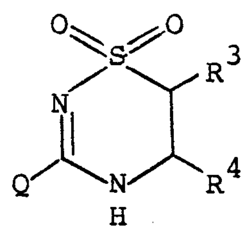

(III)

wherein $R^1$, $R^2$, $R^3$, $R^4$, Z, Y, m, n and p are as hereinbefore defined, and Q is a group displaceable by amine; or

b) reducing a compound of the formula (IV) or (V) :

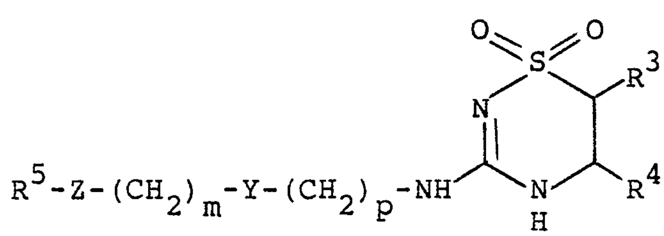

(IV)

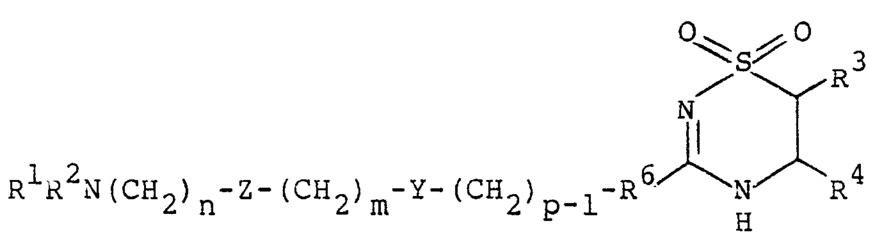

(V)

wherein $R^1$, $R^2$, $R^3$, $R^4$, Z, Y, n, m and p are as hereinbefore defined, $R^5$ is a precursor of a group $R^1R^2N(CH_2)_n$- as hereinbefore defined and $R^6$ is a group -CONH- or -CH=N-; or

c) for preparing compounds wherein Z is phenylene, m is zero and Y is oxygen, reacting a compound of the formula (VI) or chemical equivalent thereof with a compound of the formula (VII) :

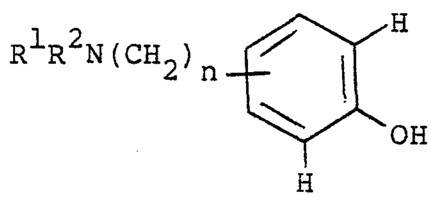

(VI)

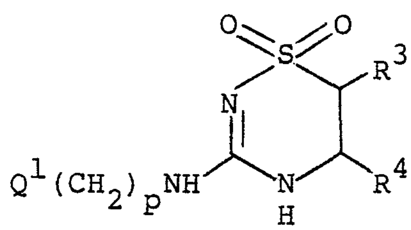

(VII)

wherein $R^1$, $R^2$, $R^3$, $R^4$, n and p are as hereinbefore defined, and $Q^1$ is a moiety displaceable by phenol or chemical equivalent thereof; or

d) for preparing compounds wherein m is one and Y is sulphur, reacting a compound of the formula (VIII) or chemical equivalent thereof with a compound of the formula (IX) :

$$R^1R^2N(CH_2)_n\text{-Z-}CH_2SH \qquad (VIII)$$

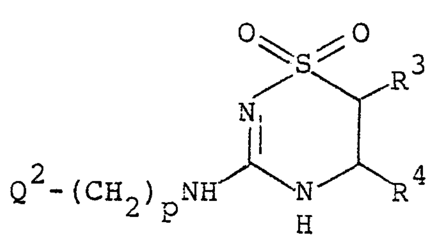

(IX)

wherein $R^1$, $R^2$, $R^3$, $R^4$, Z, n and p are as hereinbefore defined, and $Q^2$ is a moiety displaceable by thiol or chemical equivalent thereof; or

e) for preparing compounds wherein m is one and Y is sulphur, reacting a compound of the formula (X) with a compound of the formula (XI) or chemical equivalent thereof :

$$R^1R^2N(CH_2)_n-Z-CH_2-Q^3 \qquad (X)$$

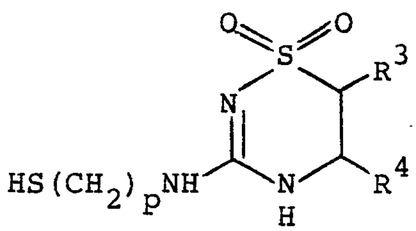

(XI)

wherein $R^1$, $R^2$, $R^3$, $R^4$, n, Z and p are as hereinbefore defined, and $Q^3$ is a moiety displaceable by thiol or chemical equivalent thereof; or

f) for preparing compounds wherein $R^3$ and $R^4$ together with the carbon atoms to which they are attached form a benzene ring optionally substituted by $C_{1-6}$alkyl, cyclizing a compound of the formula (XII) :

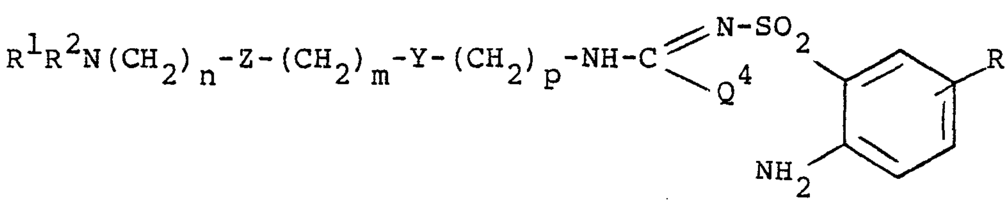

(XII)

wherein $R^1$, $R^2$, n, Z, m, Y and p are as hereinbefore defined, R is hydrogen or $C_{1-6}$alkyl, and $Q^4$ is a moiety displaceable by amine;

and optionally thereafter forming a pharmaceutically acceptable salt.

Suitably Q is $C_{1-6}$alkylthio, benzylthio, chloro or bromo. Favourably Q is methylthio.

The reaction between a compound of the formula (II) and a compound of the formula (III) can be performed in the absence of solvent at an elevated temperature, or in the presence of a substantially inert solvent such as a polar solvent, for example pyridine, anisole or a $C_{1-6}$-alkanol, at for example a temperature between ambient and reflux. When Q is methylthio the reaction for example may be performed at 120-170°C in the absence of solvent.

In the compounds of the formula (IV) in one suitable aspect $R^5$ is a group $R^1R^2N-(CH_2)_x-CO-(CH_2)_y-$ wherein $x+y = n-1$. Favourably x and y are both zero so that the group $R^1R^2NCO-$ is a precursor to the group $R^1R^2NCH_2-$. The reduction of such a group $R^1R^2N-(CH_2)_x-CO-(CH_2)_y-$ may be performed with a hydride for example lithium aluminium hydride.

In an alternative aspect $R^5$ is a group $CHO-(CH_2)_{n-1}-$, which may be converted to a group $R^1R^2N(CH_2)_n-$ on reaction with an amine $R^1R^2NH$ under conditions of reductive amination. Furthermore in another suitable aspect $R^5$ may be a group $HO(CH_2)_n-$ which may be converted directly to $R^1R^2N(CH_2)_n-$, or indirectly thereto for example via a moiety such as $Br(CH_2)_n-$ and thence to $R^1R^2N(CH_2)_n-$. Such transformations may be carried out in conventional manner.

The compounds of the formula (V) may be reduced to form compounds of the formula (I), for example using lithium aluminium hydride in an ether solvent when $R^6$ is -CONH-; and for example using a borohydride in an

alkanol, lithium aluminium hydride in an ether solvent, or catalytically hydrogenating when $R^6$ is -CH=N-.

In the reaction between the compounds of the formulae (VI) and (VII) suitably $Q^1$ is chloro or bromo. Suitably the reaction is performed under basic conditions, for example the anion of the compound of the formula (VI) may be generated, for example using sodium hydride. The reaction is performed in a suitable aprotic solvent for example dimethylformamide at a non-extreme temperature for example between 0°C and 100°C, suitably between ambient and 70°C.

Suitably in the reaction between the compounds of the formulae (VIII) and (IX) $Q^2$ is chloro, bromo, arylsulphonyloxy for example 4-methylbenzenesulphonyloxy or $C_{1-6}$alkanesulphonyloxy for example methanesulphonyloxy. Such reactions are generally performed in the presence of a base for example triethylamine, an alkoxide or a hydroxide.

Suitably in the reaction between the compounds of the formula (X) and (XI) $Q^3$ is chloro, bromo, hydroxy, $C_{1-6}$alkoxy for example methoxy, $C_{1-6}$alkanoyloxy for example acetoxy, arylsulphonyloxy for example 4-methylbenzenesulphonyloxy, or $C_{1-6}$alkanesulphonyloxy for example methanesulphonyloxy.

Preferably $Q^3$ is hydroxy in which case the reaction between the compounds of the formulae (X) and (XI) is performed under acidic conditions. When $Q^3$ is chloro or bromo it is preferable to perform the reaction in the presence of a strong base for example sodium ethoxide in ethanol. When $Q^3$ is an arylsulphonyloxy or alkylsulphonyloxy group the reaction is preferably performed under mildly basic conditions for example in pyridine solution.

The cyclisation of a compound of the formula (XII) is suitably performed in an organic solvent. Suitably $Q^4$ is a $C_{1-4}$alkylthio group for example methylthio.

Pharmaceutically acceptable acid addition salts of the compounds of the formula (I) may be prepared from the corresponding base of the compounds of the formula (I) in conventional manner. For example the base may be reacted with an acid in a $C_{1-4}$alkanol, or an ion-exchange resin may be used. The salts of the compounds of the formula (I) may be interconverted using ion-exchange resins. Non-pharmaceutically acceptable salts are therefore of use as they can be converted to pharmaceutically acceptable salts.

Suitable pharmaceutically acceptable acid addition salts of the compounds of the formula (I) include those formed with hydrochloride, hydrobromic, sulphuric, phosphoric, acetic, citric, maleic, lactic, ascorbic, fumaric, oxalic, methanesulphonic and ethanesulphonic acids.

The compounds of the formula (II), (VIII) and (X) are preparable by the methods of European Patent Application Publication Nos 13071, 49173 and 89153 or in the case of compounds of the formulae (VIII) by a modification thereof. The compounds of the formula (III) are known from UK Patent No 1419994.

The compounds of the formula (IV) may be prepared in a manner analogous to that described for the preparation of compounds of the formula (I), for example reacting a compound of the formula (III) with an analogue of a compound of the formula (II) wherein $R^1R^2N(CH_2)_n$- is replaced by $R^5$; provided that $R^5$ is suitably protected as necessary.

The compounds of the formula (V) wherein $R^6$ is CH=N may be prepared by the reaction of a compound of the formula (XIII) with a compound of the formula (XIV) :

$$R^1R^2N(CH_2)_n\text{-}Z\text{-}(CH_2)_mY(CH_2)_{p-1}CHO \qquad \text{(XIII)}$$

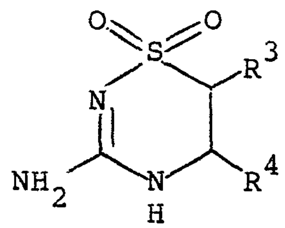

(XIV)

wherein $R^1$, $R^2$, $R^3$, $R^4$, n, m, Z, Y and p are as hereinbefore defined, optionally in the presence of an acid catalyst. The compounds of the formula (V) wherein $R^6$ is -CONH- may be prepared by the reaction of a compound of the formula (XIV) with an activated derivative of a compound of the formula (XV) :

$$R^1R^2N(CH_2)_n\text{-}Z\text{-}(CH_2)_mY(CH_2)_{p-1}CO_2H \qquad \text{(XV)}$$

wherein $R^1$, $R^2$, $R^3$, $R^4$, n, m, Z, Y and p are as hereinbefore defined. Suitable active derivatives are acyl halides, anhydrides and activated esters. The aldehydes of the formula (XIII) may be prepared for example by reacting a compound of the formula (XVI) :

$$R^1R^2N(CH_2)_n\text{-}Z\text{-}OH \qquad \text{(XVI)}$$

wherein $R^1$, $R^2$, n, and Z are as hereinbefore defined, with a protected hydroxypropionaldehyde (for example protected as a cyclic acetal) and deprotecting. The acid of the formula (XV) and derivatives thereof may be prepared in similar manner for example by reacting a compound of the formula (XVI) with a protected hydroxypropionic acid and if necessary deprotecting and/or converting to the desired activated acid derivative.

The compounds of the formula (VI) are preparable for example by the methods of Turner et al J. Org. Chem. 24 p 1952 (1959).

The compounds of the formulae (VII), (IX) and (XI) may be prepared for example by methods analogous to that described for the reaction of compounds of the formulae (II) and (III), that is reacting a compound of the formula (III) with $Q^1(CH_2)_pNH_2$, $Q^2(CH_2)_pNH_2$ or $HS(CH_2)_pNH_2$ wherein the nature of the groups $Q^1$ and $Q^2$ is such that the desired reaction occurs. The compounds of the formula (XII) for example may be prepared by the methods of BE-892350.

The following Examples serve to illustrate this invention.

## Example 1

3-[3-(3-(Piperidinomethyl)phenoxy)propylamino]-5,6-dihydro 1,2,4-thiadiazine-1,1-dioxide

3-[3-(Piperidinomethyl)phenoxy]propylamine (2.91 g) and 3-methylthio-5,6-dihydro-1,2,4-thiadiazine-1,1-dioxide (1.90 g) were combined and heated at 150-5°C for 4 hours. The melt was allowed to cool and washed with hot water. The mixture was allowed to cool and the water decanted. The residue was washed with water and washed with ether to afford the title product. This was treated with dilute ethanolic HCl to form a solid which was recrystallised from methanol-water to afford crystalline 3-[3-(3-(piperidinomethyl)phenoxy)propylamino]-5,6-dihydro-1,2,4-thiadiazine-1,1-dioxide monohydrochloride (2.94 g), m.p. 229-232°C.

Example 2

3-[3-(3-(Piperidinomethyl)phenoxy)propylamino]-1,2,4-benzothiadiazine-1,1-dioxide

3-[3-(Piperidinomethyl)phenoxy]propylamine (1.24 g) and 3-methylthio-1,2,4-benzothiadiazine-1,1-dioxide (1.14 g) were fused at 140-150°C for 2 hours. The mixture was cooled, dissolved in isopropanol, and evaporated under reduced pressure to afford a residue. This residue was left standing with diethyl ether for 16 hours at 0°C, to give a white solid which was recrystallised from isopropanol to afford the title product in crystalline form, m.p. 147-148°C.

Example 3

3-[2-(4-Dimethylaminomethylpyrid-2-ylmethylthio)ethylamino]-5,6-dihydro-1,2,4-thiadiazine-1,1-dioxide

2-(4-Dimethylaminomethylpyrid-2-ylmethylthio)ethylamine (0.9 g) and 3-methylthio-5,6-dihydro-1,2,4-thiadiazine-1,1-dioxide (0.9 g) were fused at 130-140°C for 2 hours. The mixture was cooled, washed with diethyl ether and subjected to chromatography on silica gel (h.p.l.c., 250 mm x 15 mm column eluting with 100% $CH_2Cl_2$ grading to 95% $CH_2Cl_2$/5% $CH_3OH$). The desired fractions were collected and evaporated under reduced pressure to afford the title compound as an oil (0.7 g). This was treated with oxalic acid in ethanol to give a solid which was recrystallised from methanol to afford crystalline 3-[2-(4-dimethylaminomethylpyrid-2-ylmethylthio)ethylamino]-5,6-dihydro-1,2,4-thiadiazine-1,1-dioxide dioxalate (0.67 g), m.p. 177-8°C.

## Example 4

**3-[2-(4-Dimethylaminomethylpyrid-2-ylmethylthio)ethylamino]-1,2,4-benzothiadiazine-1,1-dioxide**

2-(4-Dimethylaminomethylpyrid-2-ylmethylthio)ethylamine (1.0 g) and 3-methylthio-1,2,4-benzothiadiazine-1,1-dioxide (1.0 g) were fused at 140°C for 2 hours. The reaction mixture was cooled to afford the title compound as a crude residue, which was dissolved in ethanol and treated with oxalic acid (2.0 g) to afford crystals. These were recrystallised from methanol to afford 3-[2-(4-dimethylaminomethylpyrid-2-ylmethylthio)ethylamino]-1,2,4-benzothiadiazine-1,1-dioxide dioxalate (1.2 g), m.p. 157-8°C.

## Example 5

**3-[3-(4-Dimethylaminomethylpyrid-2-yloxy)propylamino]-1,2,4-benzothiadiazine-1,1-dioxide**

3-(4-Dimethylaminomethylpyrid-2-yloxy)propylamine (1.1 g) and 3-methylthio-1,2,4-benzothiadiazine-1,1-dioxide (1.0 g) were fused at 140-150°C for 2 hours. The reaction mixture was cooled to afford the title compound as a crude residue, which was dissolved in ethanol (5 ml) and treated with oxalic acid (1.0 g) in ethanol (5 ml) to afford a solid. This solid was recrystallised from methanol to afford crystalline 3-[3-(4-dimethylaminomethylpyrid-2-yloxy)propylamino]-1,2,4-benzothiadiazine-1,1-dioxide dioxalate, m.p. 181-2°C.

## Example 6

3-[3-[4-Piperidinomethylpyrid-2-yloxy]propylamino]-5,6-dihydro-1,2,4-thiadiazine-1,1-dioxide

3-[4-Piperidinomethylpyrid-2-yloxy]propylamine (1.2 g) and 3-methylthio-5,6-dihydro-1,2,4-thiadiazine-1,1-dioxide (0.9 g) were heated at 140°C for 2 hours. The reaction mixture was cooled to afford the title compound as a pale orange oil. This was treated with oxalic acid in ethanol to afford solid which was recrystallised from ethanol to give crystalline 3-[3-[4-piperidinomethylpyrid-2-yloxy]propylamino]-5,6-dihydro-1,2,4-thiadiazine-1,1-dioxide dioxalate (0.51 g), m.p. 155-6°C.

## Example 7

3-[3-[4-Piperidinomethylpyrid-2-yloxy]propylamino]-1,2,4-benzothiadiazine-1,1-dioxide

3-[4-Piperidinomethylpyrid-2-yloxy]propylamine (1.2 g) and 3-methylthio-1,2,4-benzothiadiazine-1,1-dioxide (1.1 g) were heated at 145°C for 2 hours. The reaction mixture was cooled to afford the title compound as a crude residue. This residue was dissolved in ethanol and treated with oxalic acid (2 g) to afford a solid. This solid was recrystallised from methanol to give 3-[3-[4-piperidinomethylpyrid-2-yloxy]propylamino]-1,2,4-benzothiadiazine-1,1-dioxide dioxalate (1.32 g), m.p. 186-7°C.

## Example 8

A pharmaceutical composition for oral administration is prepared containing:

| | | % by weight |
|---|---|---|
| A | 3-[3-[4-piperidinomethylpyrid-2-yloxy]-propylamino]-1,2,4-benzothiadiazine-1,1-dioxide dioxalate | 55 |
| A | Dibasic calcium phosphate dihydrate | 20 |
| A | Approved colouring agent | 0.5 |
| | Polyvinylpyrrolidone | 4.0 |
| B | Microcrystalline Cellulose | 8.0 |
| B | Maize Starch | 8.0 |
| B | Sodium glycollate | 4.0 |
| B | Magnesium Stearate | 0.5 |

by mixing together the ingredients A (substituting lactose or microcrystalline cellulose for dibasic calcium phosphate dihydrate if desired), adding a concentrated solution of polyvinylpyrrolidone and granulating, drying and screening the dried granules; adding the ingredients B to the dried granules and compressing the mixture into tablets containing 100 mg, 150 mg or 200 mg of the free base.

Other compounds of the invention, for example those specifically described in Examples 1 to 6 can be formulated into pharmaceutical compositions by a similar procedure.

The compounds of this invention, where tested, show no overt signs of toxicity at doses which are a pertinent multiple of the therapeutic dose.

CLAIMS :

1. A compound of the formula (I) :-

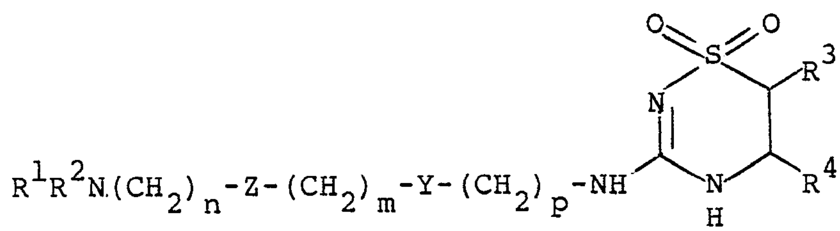

(I)

or a pharmaceutically acceptable salt thereof, wherein

$R^1$ and $R^2$ are independently hydrogen, $C_{1-6}$alkyl, aryl($C_{1-6}$)alkyl, heteroaryl($C_{1-6}$)alkyl, $C_{3-10}$cyclo-alkyl, hydroxy($C_{2-6}$)alkyl, or halo-($C_{2-6}$)alkyl, (wherein said hydroxy and halo groups are not substituted on the carbon atom adjacent to the nitrogen atom); or

$R^1$ and $R^2$ together represent $-(CH_2)_q-$ wherein q is 4 to 7, to form together with the nitrogen atom to which they are attached a 5-8 membered saturated ring;

n is an integer from 1 to 6;

Z is 1,3-phenylene, 1,4-phenylene, 2,4-pyridyl (wherein the $R^1R^2N(CH_2)_n$ group is in the 4-position);

m is zero or one ;

Y is oxygen, sulphur or methylene;

p is two, three or four; and

$R^3$ and $R^4$ are independently hydrogen, $C_{1-6}$alkyl, aryl, aryl($C_{1-6}$)alkyl or pyridyl($C_{1-6}$)alkyl, or

$R^3$ and $R^4$ together with the carbon atoms to which they are attached form a benzene ring optionally substituted by $C_{1-6}$alkyl.

2. A compound according to claim 1 wherein $R^3$ and $R^4$ are independently hydrogen, $C_{1-6}$alkyl, aryl, aryl-($C_{1-6}$)alkyl or pyridyl($C_{1-6}$)alkyl.

3. A compound according to claim 2 wherein $R^3$ and $R^4$ are both hydrogen.

4. A compound according to any one of claims 1 to 3 wherein Z is 2,4-pyridyl.

5. A compound according to any one of claims 1 to 3 wherein Z is 1,3-phenylene.

6. A compound according to any one of claims 1 to 5 wherein $R^1R^2N(CH_2)_n$- is piperidinomethyl.

7. A compound according to any one of claims 1 to 6 wherein m is zero and Y is oxygen.

8. A compound according to claim 1 which is :

3-[3-(3-(piperidinomethyl)phenoxy)propylamino]-5,6-dihydro-1,2,4-thiadiazine-1,1-dioxide or a pharmaceutically acceptable salt thereof;

3-[3-(3-(piperidinomethyl)phenoxy)propylamino]-1,2,4-benzo thiadiazine-1,1-dioxide or a pharmaceutically acceptable salt thereof;

3-[3-(4-(dimethylaminomethyl)pyrid-2-yloxy)propylamino]-1,2,4-benzothiadiazine-1,1-dioxide or a pharmaceutically acceptable salt thereof;

3-[3-(4-(piperidinomethyl)pyrid-2-yloxy)propylamino]-1,2,4-benzothiadiazine-1,1-dioxide or a pharmaceutically acceptable salt thereof;

3-[3-(4-(piperidinomethyl)pyrid-2-yloxy)propylamino]-5,6-dihydro-1,2,4-thiadiazine-1,1-dioxide or a pharmaceutically acceptable salt thereof;

3-[2-(4-(dimethylaminomethyl)pyrid-2-ylmethylthio)ethylamino]-1,2,4-benzothiadiazine-1,1-dioxide or a pharmaceutically acceptable salt thereof; or

3-[2-(4-(dimethylaminomethyl)pyrid-2-ylmethylthio)ethylamino]-5,6-dihydro-1,2,4-thiadiazine-1,1-dioxide or a pharmaceutically acceptable salt thereof.

9. Crystalline 3-[3-(3-(piperidinomethyl)phenoxy)-propylamino]-1,2,4-benzothiadiazine-1,1-dioxide.

10. A compound according to any one of claims 1 to 9 for use as a therapeutic agent.

11. A compound according to any one of claims 1 to 9 for use as an $H_2$-antagonist.

12. A pharmaceutical composition which comprises a compound according to any of claims 1 to 9 and a pharmaceutically acceptable carrier.

13. A process for preparing a compound of the formula (I) as defined in claim 1 or a pharmaceutically acceptable salt thereof which comprises :

a) reacting a compound of the formula (II) with a compound of the formula (III) :

$$R^1R^2N(CH_2)_n-Z-(CH_2)_m-Y-(CH_2)_p-NH_2 \qquad (II)$$

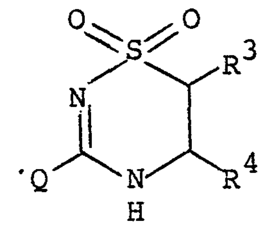

(III)

wherein $R^1$, $R^2$, $R^3$, $R^4$, Z, Y, m, n and p are as defined in claim 1, and Q is a group displaceable by amine; or

b) reducing a compound of the formula (IV) or (V) :

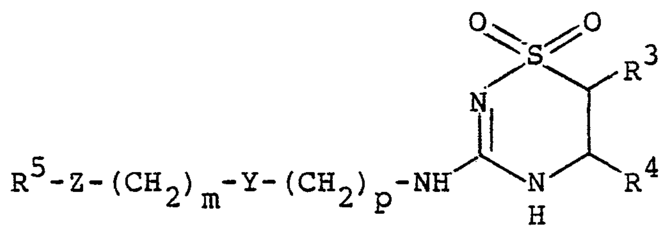

(IV)

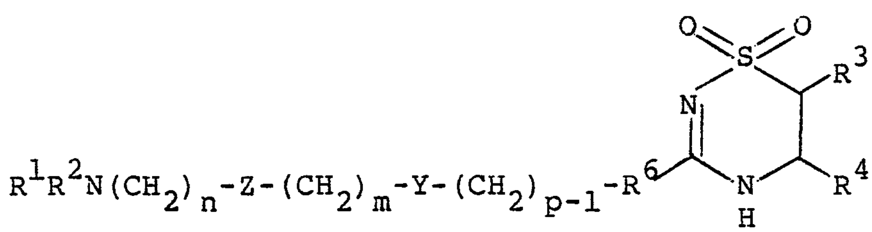

(V)

wherein $R^1$, $R^2$, $R^3$, $R^4$, Z, Y, n, m and p are as hereinbefore defined, $R^5$ is a precursor of a group $R^1R^2N(CH_2)_n-$ as hereinbefore defined and $R^6$ is a group -CONH- or -CH=N-; or

c) for preparing compounds wherein Z is phenylene, m is zero and Y is oxygen, reacting a compound of the formula (VI) or chemical equivalent thereof with a compound of the formula (VII) :

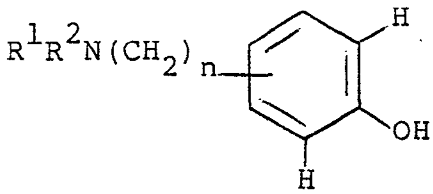

(VI)

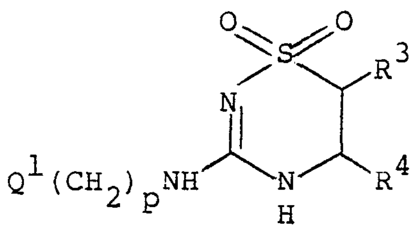

(VII)

wherein $R^1$, $R^2$, $R^3$, $R^4$, n and p are as hereinbefore defined, and $Q^1$ is a moiety displaceable by phenol or chemical equivalent thereof; or

d) for preparing compounds wherein m is one and Y is sulphur, reacting a compound of the formula (VIII) or chemical equivalent thereof with a compound of the formula (IX) :

$$R^1R^2N(CH_2)_n\text{-}Z\text{-}CH_2SH \qquad \text{(VIII)}$$

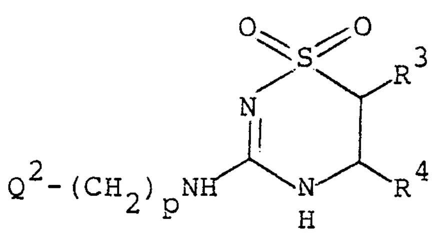

(IX)

wherein $R^1$, $R^2$, $R^3$, $R^4$, Z, n and p are as hereinbefore defined, and $Q^2$ is a moiety displaceable by thiol or chemical equivalent thereof; or

e) for preparing compounds wherein m is one and Y is sulphur, reacting a compound of the formula (X) with a

compound of the formula (XI) or chemical equivalent thereof :

$$R^1R^2N(CH_2)_n\text{-}Z\text{-}CH_2\text{-}Q^3 \qquad (X)$$

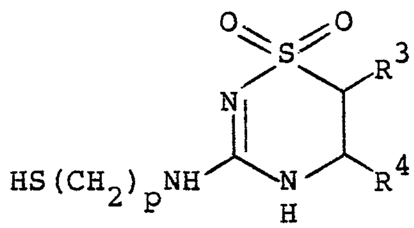

(XI)

wherein $R^1$, $R^2$, $R^3$, $R^4$, n, Z and p are as hereinbefore defined, and $Q^3$ is a moiety displaceable by thiol or chemical equivalent thereof; or

f) for preparing compounds wherein $R^3$ and $R^4$ together with the carbon atoms to which they are attached form a benzene ring optionally substituted by $C_{1-6}$alkyl, cyclizing a compound of the formula (XII) :

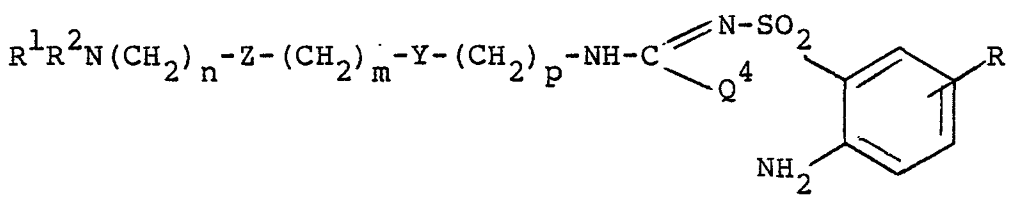

(XII)

wherein $R^1$, $R^2$, n, Z, m, Y and p are as hereinbefore defined, R is hydrogen or $C_{1-6}$alkyl, and $Q^4$ is a moiety displaceable by amine;

and optionally thereafter forming a pharmaceutically acceptable salt.

Claims for Austria

1. A process for preparing a compound of the formula (I) :-

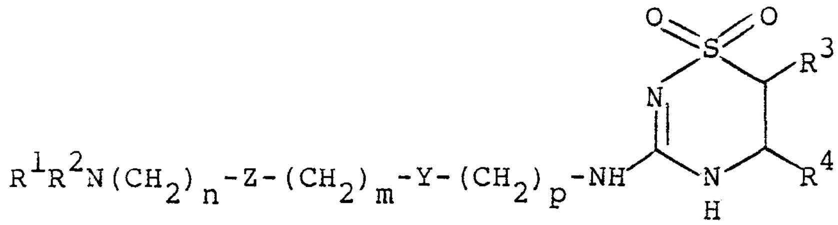

(I)

or a pharmaceutically acceptable salt thereof, wherein

$R^1$ and $R^2$ are independently hydrogen, $C_{1-6}$alkyl, aryl($C_{1-6}$)alkyl, heteroaryl($C_{1-6}$)alkyl, $C_{3-10}$cycloalkyl, hydroxy($C_{2-6}$)alkyl, or halo-($C_{2-6}$)alkyl, (wherein said hydroxy and halo groups are not substituted on the carbon atom adjacent to the nitrogen atom); or

$R^1$ and $R^2$ together represent $-(CH_2)_q-$ wherein q is 4 to 7, to form together with the nitrogen atom to which they are attached a 5-8 membered saturated ring;

n is an integer from 1 to 6;

Z is 1,3-phenylene, 1,4-phenylene, 2,4-pyridyl (wherein the $R^1R^2N(CH_2)_n$ group is in the 4-position);

m is zero or one ;

Y is oxygen, sulphur or methylene;

p is two, three or four; and

$R^3$ and $R^4$ are independently hydrogen, $C_{1-6}$alkyl, aryl, aryl($C_{1-6}$)alkyl or pyridyl($C_{1-6}$)alkyl, or $R^3$ and $R^4$ together with the carbon atoms to which

they are attached form a benzene ring optionally substituted by $C_{1-6}$alkyl;

which process comprises :

a) reacting a compound of the formula (II) with a compound of the formula (III) :

$$R^1R^2N(CH_2)_n-Z-(CH_2)_m-Y-(CH_2)_p-NH_2 \quad (II)$$

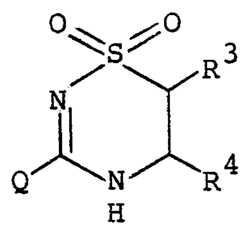

(III)

wherein $R^1$, $R^2$, $R^3$, $R^4$, Z, Y, m, n and p are as defined in claim 1, and Q is a group displaceable by amine; or

b) reducing a compound of the formula (IV) or (V) :

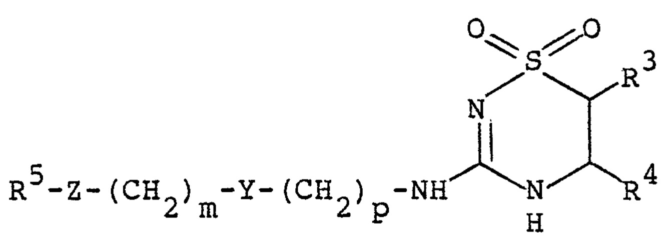

(IV)

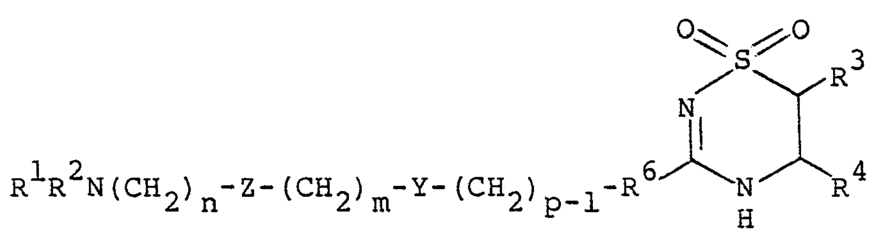

(V)

wherein $R^1$, $R^2$, $R^3$, $R^4$, Z, Y, n, m and p are as hereinbefore defined, $R^5$ is a precursor of a group

$R^1R^2N(CH_2)_n$- as hereinbefore defined and $R^6$ is a group -CONH- or -CH=N-; or

c) for preparing compounds wherein Z is phenylene, m is zero and Y is oxygen, reacting a compound of the formula (VI) or chemical equivalent thereof with a compound of the formula (VII) :

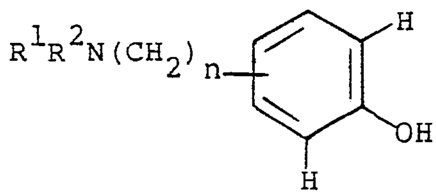

(VI)

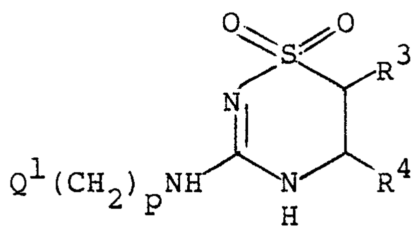

(VII)

wherein $R^1$, $R^2$, $R^3$, $R^4$, n and p are as hereinbefore defined, and $Q^1$ is a moiety displaceable by phenol or chemical equivalent thereof; or

d) for preparing compounds wherein m is one and Y is sulphur, reacting a compound of the formula (VIII) or chemical equivalent thereof with a compound of the formula (IX) :

$$R^1R^2N(CH_2)_n\text{-}Z\text{-}CH_2SH \quad \text{(VIII)}$$

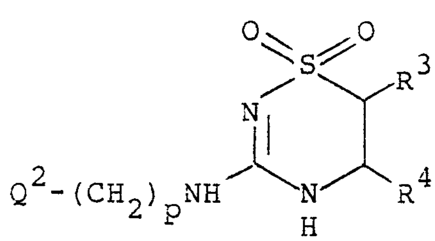

(IX)

wherein $R^1$, $R^2$, $R^3$, $R^4$, Z, n and p are as hereinbefore defined, and $Q^2$ is a moiety displaceable by thiol or chemical equivalent thereof; or

e) for preparing compounds wherein m is one and Y is sulphur, reacting a compound of the formula (X) with a compound of the formula (XI) or chemical equivalent thereof :

$$R^1R^2N(CH_2)_n-Z-CH_2-Q^3 \qquad (X)$$

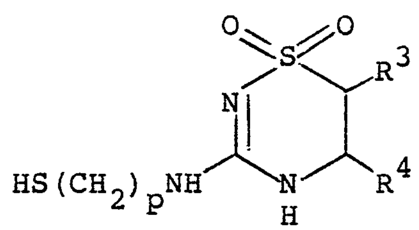

(XI)

wherein $R^1$, $R^2$, $R^3$, $R^4$, n, Z and p are as hereinbefore defined, and $Q^3$ is a moiety displaceable by thiol or chemical equivalent thereof; or

f) for preparing compounds wherein $R^3$ and $R^4$ together with the carbon atoms to which they are attached form a benzene ring optionally substituted by $C_{1-6}$alkyl, cyclizing a compound of the formula (XII) :

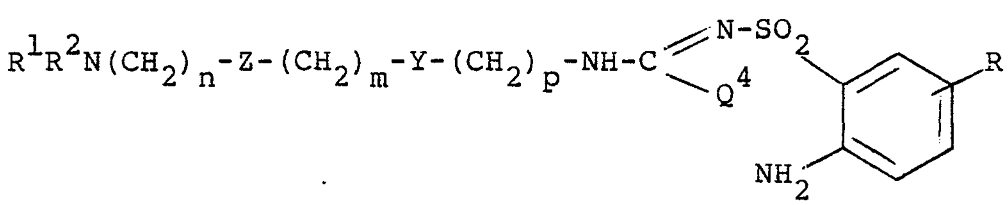

(XII)

wherein $R^1$, $R^2$, n, Z, m, Y and p are as hereinbefore defined, R is hydrogen or $C_{1-6}$alkyl, and $Q^4$ is a moiety displaceable by amine;

and optionally thereafter forming a pharmaceutically acceptable salt.

2. A process according to claim 1 for preparing a compound wherein $R^3$ and $R^4$ are independently hydrogen, $C_{1-6}$alkyl, aryl, aryl($C_{1-6}$)alkyl or pyridyl($C_{1-6}$)alkyl.

3. A process according to claim 2 for preparing a compound wherein $R^3$ and $R^4$ are both hydrogen.

4. A process according to any one of claims 1 to 3 for preparing a compound wherein Z is 2,4-pyridyl.

5. A process according to any one of claims 1 to 3 for preparing a compound wherein Z is 1,3-phenylene.

6. A process according to any one of claims 1 to 5 for preparing a compound wherein $R^1R^2N(CH_2)_n$- is piperidinomethyl.

7. A process according to any one of claims 1 to 6 for preparing a compound wherein m is zero and Y is oxygen.

8. A process according to claim 1 for preparing :

3-[3-(3-(piperidinomethyl)phenoxy)propylamino]-5,6-dihydro-1,2,4-thiadiazine-1,1-dioxide or a pharmaceutically acceptable salt thereof;

3-[3-(3-(piperidinomethyl)phenoxy)propylamino]-1,2,4-benzo thiadiazine-1,1-dioxide or a pharmaceutically acceptable salt thereof;

3-[3-(4-(dimethylaminomethyl)pyrid-2-yloxy)propylamino]-1,2,4-benzothiadiazine-1,1-dioxide or a pharmaceutically acceptable salt thereof;

3-[3-(4-(piperidinomethyl)pyrid-2-yloxy)propylamino]-1,2,4-benzothiadiazine-1,1-dioxide or a pharmaceutically acceptable salt thereof;

3-[3-(4-(piperidinomethyl)pyrid-2-yloxy)propylamino]-5,6-dihydro-1,2,4-thiadiazine-1,1-dioxide or a pharmaceutically acceptable salt thereof;

3-[2-(4-(dimethylaminomethyl)pyrid-2-ylmethylthio)ethylamino]-1,2,4-benzothiadiazine-1,1-dioxide or a pharmaceutically acceptable salt thereof; or

3-[2-(4-(dimethylaminomethyl)pyrid-2-ylmethylthio)ethylamino]-5,6-dihydro-1,2,4-thiadiazine-1,1-dioxide or a pharmaceutically acceptable salt thereof.

9. A process for preparing a pharmaceutical composition which comprises bringing into association a compound as defined in claim 1 and a pharmaceutically acceptable carrier.

European Patent Office

# EUROPEAN SEARCH REPORT

0172968

EP 84 30 7285

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | GB-A-1 604 674 (ALLEN & HANBURYS) * Whole document and in particular claim 18 * | 1-13 | C 07 D 285/18<br>C 07 D 417/12<br>A 61 K 31/54 |
| D,Y | US-A-3 932 644 (G.J. DURANT) * Whole document and in particular example 18 * | 1-13 | |
| | | | **TECHNICAL FIELDS SEARCHED (Int. Cl.4)** |
| | | | C 07 D 285/00<br>C 07 D 417/00 |

The present search report has been drawn up for all claims

| Place of search | Date of completion of the search | Examiner |
|---|---|---|
| THE HAGUE | 23-01-1985 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82